# EUROPEAN PATENT APPLICATION

(11) **EP 1 374 987 A1**
(43) Date of publication of application: **02.01.2004**
(21) Application number: 03076762.8
(22) Date of filing: 06.06.2003
(51) Int. Cl.: B01J 19/00, G01N 33/58

(54) **Micro-array identification**

(30) Priority: 18.06.2002 US 174190
(71) Applicant: EASTMAN KODAK COMPANY, Rochester, New York 14650 (US)
(72) Inventor: Kocher, Thomas E., Rochester, New York 14650-2201 (US)
(74) Representative: Weber, Etienne Nicolas

(57) **Abstract**

Micro-array receiver apparatus comprising : a micro-array receiver including a substrate and at least a first set of sites of a first biological probe which is adapted to interact with a first biological target sample, the first set of sites formed on the substrate; and an identifier associated with the micro-array receiver for identifying the at least first biological probe.

## Description

This invention relates in general to molecular biological systems and more particularly to a means by which micro-array receivers can be identified.

There is a need to identify micro-array receivers or substrates of molecular biological reagents and samples so that both automated machines as well as humans can identify the micro-array receiver. As is well known (and described for example in U.S. Patent No. 5,807,522 to Brown et al., issued Sep. 15, 1998, and in "DNA Microarrays: A Practical Approach", Schena, Mark, New York, Oxford University Press, 1999, ISBN 0-19-963776-8), micro-arrays are arrays of very small samples of purified DNA or protein probe material arranged as a grid of hundreds or thousands of small spots on a substrate. When the micro-array is exposed to unknown target material, the target material selectively binds to the probe spots only where complementary bonding sites occur, through a process called hybridization. Subsequent quantitative scanning in a fluorescent micro-array scanner may be used to produce a pixel map of fluorescent intensities (See e.g., U.S. Patent No. 5,895,915, DeWeerd et al., issued Apr. 20, 1999). This fluorescent intensity map can then be analyzed by special purpose algorithms that reveal the relative concentrations of the fluorescent targets and hence the level of gene expression, protein concentration, etc., present in the cells from which the target samples were extracted. In laboratory practice, a micro-array exposed to a sample target material, scanned and mapped via bio-informatic specific algorithms can be repeated many times with different arrays containing different probe materials and for different samples.

The present invention provides improved system productivity for the production of arrays of molecules on rigid, semi-rigid or flexible supports. Historically, arrays could be constructed either manually or mechanically through the use of photolithographic, robotically controlled or other apparatus for the precise metering and placement of molecules. Alternatively, arrays could be constructed through direct chemical synthesis on a solid support. Such devices and methods have the undesirable result that micro-arrays with a great number of individual molecular biological reagents are contained with little or no means to identify them uniquely, either by human observations or machine. The present invention addresses the drawbacks of previous systems and methods, by providing a means for the identification of arrays of molecules on rigid, semi-rigid or flexible supports.

Many examples exist for dispensing liquids in small volumes in the range of milliliters to sub-fractions of milliliters. For example, Pastinen et al. (Genorne Research, 7, 606-614 (1997)) create an array of oligonucleotides by manually applying 0.5 ~IL of a solution of 5' -amino-modified oligonucleotides onto an epoxide-activated glass slide to produce a 3x3 array of oligonucleotides on a 0.36 cm~ area of a preprinted glass slide.

Other, more traditional printing methods have been used to create patterns of a few different reagents on a solid support. Means such as silk screening, offset printing, and rotogravure printing have been used in the production of reagent test strips. In such methods, each reagent ink in applied separately. Johnson for example, U.S. Patent No. 4,216,245, issued Aug. 5, 1980, discusses methods for the production of reagent test strip devices.

Pipette dispensing of reagents can be automated. Automation potentially increases the speed and accuracy of array production, while decreasing the necessary spacing between array positions. However, the utility of automated pipetting methods are severely limited in the number of different reagents that may be simultaneously applied (low parallelism). Cozzette et al., for example, U.S. Patent No. 5,554,339, issued Sep. 10, 1996, discusses the use of micro-syringes for dispensing reagents during the production of bio-sensor devices.

High-speed robotics have also been used to print micro-arrays of amino-modified cDNA molecules onto silylated glass microscope slides (CEL Associates, Houston) or poly-1-lysine coated microscope slides (Schena, Bioassays 18:427-431 (1996); Schena et al., Proc. National. Academy Science, USA, 93:10614-10619 (1996).

Another approach to array printing is an adaptation of ink-jetting technology. For example, Hayes et al., U.S. Patent No. 4,877,745, issued Oct. 31, 1989, discusses an ink-jet type method and apparatus for dispensing reagents, particularly in the production of reagent test strips.

Pin transfer is one approach that allows the simultaneous transfer of greater numbers of samples than possible with the above approaches.

Pirrung et al., U.S. Patent No. 5,143,854, issued Sep. 1, 1992, Fodor et al., U.S. Patent No. 5,510,270, issued Apr. 23, 1996, Fodor et al., U.S. Patent No. 5,445,934, issued Aug. 29, 1995, and Chee et al., WO95/11995, issued May 4, 1995, discusses the production of high 2 density oligonucleotide arrays through photolithographic synthesi's of thd'oligonucle-otides, directly onto derivatized glass substrate.

McGall et al., U.S. Patent No. 5,412,087, issued May 2, 1995, discusses a method for the production of a high density oligonucleotide array from pre-synthesized oligonucleotides.

Birch et al., U.S. Patent No. 6,051,190 , issued Apr. 18, 2000, and U.S. Patent No. 6,303,387, issued Oct. 16, 2001, discusses a transfer rod for distribution of small amounts of liquid in biological or chemical analysis.

Bryning et al., U.S. Patent No. 6,296,702 B1, issued Oct. 2, 2001, discusses an oscillating fiber apparatus for dispensing small volumes of a selected liquid onto a substrate.

Kowallis, U.S. Patent No. 6,245,297 B1, issued Jun. 12, 2001, discusses an apparatus with a transport pathway for the production of micro-arrays having a great number of closely spaced spots.

In view of the above, the need is apparent for an efficient means for identifying the molecular biological reagents and samples that are contained on solid or semi-solid supports as well as any applications of additional materials or modifications to the micro-array through use in a laboratory environment. Preferably, the means should be relatively easy to implement, robust, and provide both a machine as well as a human identifiable piece and readily applicable to the production of micro-arrays having a great number of individual spots. Additionally, the means should be able to modify or update the information content contained in the identifying means thereby providing a method by which every step can be tracked through the processes of - array manufacturing, sample application, and finally scanning/readout.

According to the present invention, there is provided a solution to the problems and fulfillment of the needs discussed above.

According to a feature of the present invention, there is provided a micro-array receiver apparatus comprising: a micro-array receiver including a substrate and at least a first set of sites of a first biological probe which is adapted to interact with a first biological target sample, said first set of sites formed on said substrate; and
an identifier associated with said micro-array receiver for identifying said at least first biological probe.

The invention has the following advantages.
1. A micro-array receiver is provided having an identifier for identifying the biological probe(s) formed on said receiver.
2. The identifier is relatively easy to implement, is robust, and provides an identifier which can be both human and machine readable.
3. The identifier can be a type (magnetic, RFID) which is modifiable so that information can be modified, added or deleted.

Fig. 1 is a diagrammatic view of an embodiment of the present invention.
Fig. 2 is a diagrammatic view of another embodiment of the present invention.
Fig. 3 is a block diagram of a workflow which can be used with the present invention.
Fig. 4 is a diagrammatic view of details of an exemplary micro-array receiver which can be used in the present invention.

In general, the present invention relates to micro-array receiver apparatus having a micro-array receiver including a substrate and at least a first biological probe which is adapted to interact with a first target sample. The sites are formed on the substrate. An identifier is associated with the micro-array receiver for identifying the first biological probe. Preferably the micro-array receiver includes a plurality of sets of sites each set of sites being of a different biological probe which is adapted to interact with a different biological target sample. The plurality of sets of sites are formed on the substrate and the identifier identifies each of the plurality of biological probes.

The identifier can also identify one or more of the following: the target biological samples which interact with the biological probe(s); information about the micro-array, how it was manufactured, and how it was used in its application; information that can be modified, updated during the manufacture, sample application/readout processes.

The identifier can be one or more of the following; human readable information; bar code; magnetic or optical strip; radio-frequency identification (RFID) tag; etc.

The biological target site(s) formed on the micro-array receiver substrate can be formed by one or more of the following techniques: photolithography; ink jet printing, silk screening, liquid dispensing.

Preferably the micro-array receiver includes a substrate coated with a composition comprising micro-spheres (beads) dispersed in a fluid containing a gelling agent or a precursor to a gelling agent, wherein the micro-spheres are immobilized in a random or ordered position on the substrate. The substrate is free of receptors designed to physically or chemically interact with the micro-spheres. One or more sub-populations of the population of micro-spheres contain a unique optical bar code generated from at least one colorant associated with the micro-spheres and including a unique biological functionality or probe which reacts with analytes with which they come in contact.

The distribution or pattern of micro-spheres on the substrate may be entirely random (a spatial distribution showing no reference or bias) or be attracted or held to sites that are pre-marked or predetermined on the substrate. Each micro-sphere in the array has a distinct signature based on color which may be derived from mixing three dyes representing the primary colors Red (R), Green (G), and Blue (B) to create thousands of distinguishable micro-spheres with distinct color addresses (unique RGB values, e.g., R=0, G=204, B=153). The micro-spheres are made with active sites on their surface to which are attached a specific bioactive probe. Therefore, each color address can correspond to a specific bioactive probe.

A micro-array or population of micro-spheres can include a few or hundreds or more of sub-populations of micro-spheres, where each sub-population comprises the same color code and the same bio-active probe. Each micro-array of micro-spheres occupies a sub-area of the substrate and is repeated in a pattern over the area of the substrate. The dimensional area of the micro-array sub-area may be comparable to the dimensional area of a microtiter well or multiple wells may overlay a micro-array sub-area.

The micro-spheres are preferably coated onto the substrate as disclosed in U.S. Patent Application S.N. 09/942,241, chari et al.

In order to use a micro-array having bioactive probes to analyze an unknown biological target sample, the sample to be analyzed has to be non-selectively labeled by using fluorescent dyes or chemiluminescent active molecules.

A biological target sample is placed into contact with the micro-array bioactive probes. The fluorescently/chemiluminescently signals which result from the hybridization of the unknown biological target sample with bioactive probes on the surface of the coated micro-spheres are detected and analyzed by an electronic camera/image processor system.

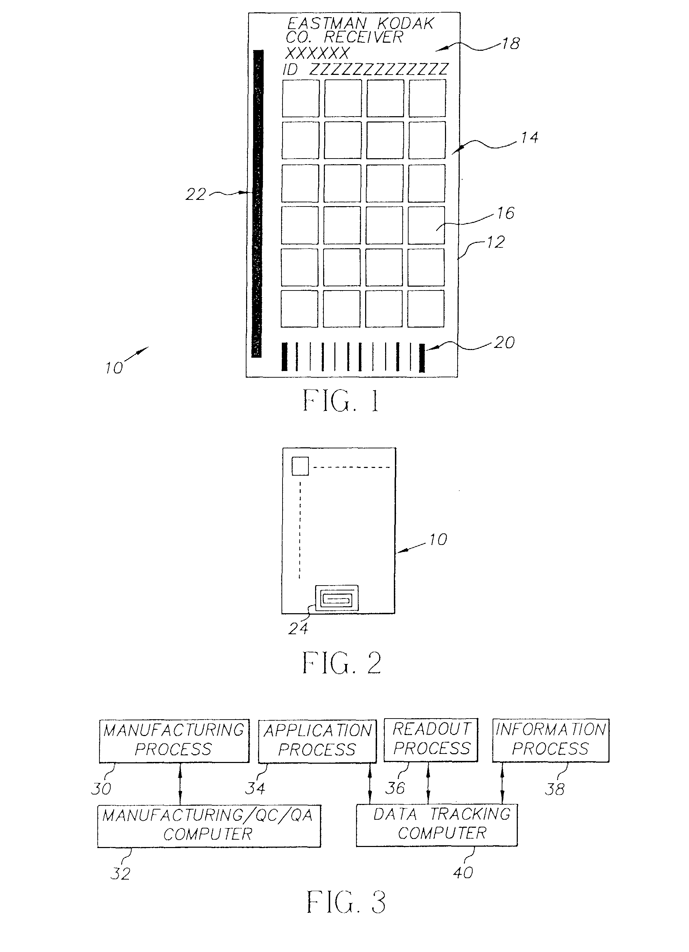

Referring to Fig. 1, there is shown micro-array receiver apparatus **10** according to the present invention. Apparatus **10** includes a micro-array receiver **12** including a substrate **14** having formed thereon a pattern of twenty-four regions **16** in a matrix of four rows and six columns (as viewed by rotating Fig. 1 by 90°). Each region includes an identical micro-array of a plurality of biological probe sites. Each different biological probe is adapted to interact with a specific biological target sample.

Micro-array receiver apparatus **10** includes several identifiers **18**, **20** and **22** for storing or presenting information relative to the micro-array receiver and its biological probes. Identifier **18** is human readable including letters, numbers and other visually identifiable symbols. Identifier **20** is a machine readable bar code. Identifier **22** is a magnetic strip that stores information that can be updated (dynamically) to reflect the state of manufacturing and how it was used. Human readable identifier **18** can identify the specific biological activity associated with the receiver. It could also identify that a receiver is one of a number of receivers in a specific series (e.g., "3 of 10"). Bar code identifier **20** can be read by the manufacturing computers as well as by a data tracking computer of the end user.

Fig. 2 shows apparatus **10** with an RFID identifier **24** which can be written to and read from using well known RFID technology.

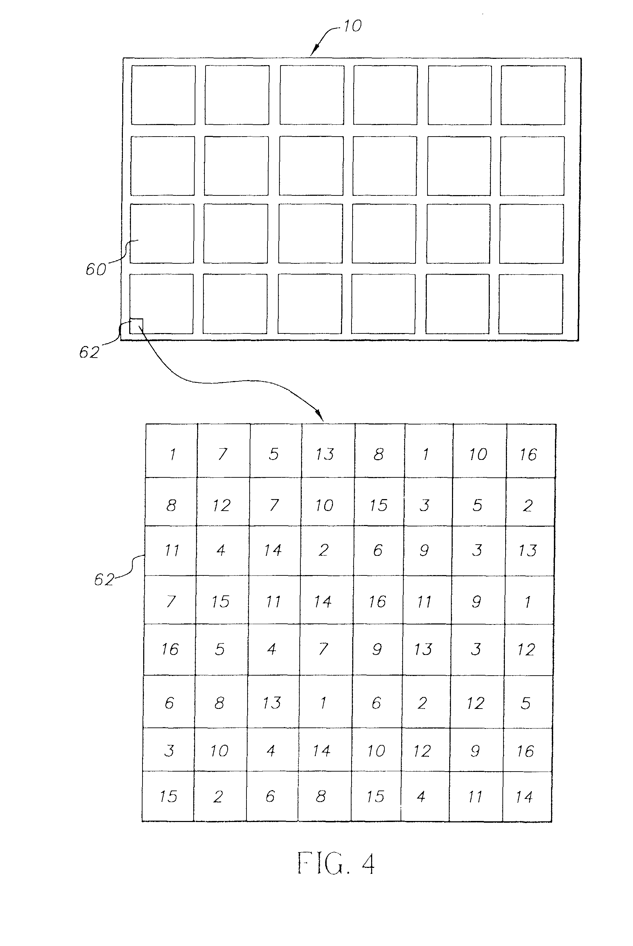

Fig. 3 illustrates the functions of an identifier **22** which can be updated. Box **30** represents a manufacturing process that can be stored on magnetic strip **22** or RFID identifier **24** and which can be changed by a manufacturing/QC/QA computer **32**. Boxes **34**, **36**, and **38** represent application process, readout process and informatics process that can be stored in a magnetic or RFID identifier and which can be updated by data tracking computer **40**.

Fig. 4 illustrates an example of micro-array receiver apparatus **10** including regions **60** having identical micro-array of randomly distributed biological probe sites, a portion **62** of which are shown in the exploded view. In this view, sixteen different biological probes attached to micro-spheres, are randomly distributed throughout portion **62** of region **60**. Each probe is attached to a micro-sphere of a color unique to that probe, so that micro-spheres of sixteen different colors are present in portion **16**. If, for example, a sample analyte containing each of the sixteen complementary targets to the sixteen probes is brought into contact with portion **16**, the hybridization between the sixteen targets and sixteen probes would produce luminescence or fluorescence of sixteen different colors which can be detected by an appropriate detection system.

## Claims

1. Micro-array receiver apparatus comprising:
a micro-array receiver including a substrate and at least a first set of sites of a first biological probe which is adapted to interact with a first biological target sample, said first set of sites formed on said substrate; and
an identifier associated with said micro-array receiver for identifying said at least first biological probe.

2. The micro-array receiver apparatus of claim 1 wherein said identifier is one or more of a bar code, a human readable identifier, a magnetic region, a radio frequency identifier.

3. The micro-array receiver apparatus of claim 1 wherein said micro-array receiver includes a plurality of sets of sites, each set of sites being of a different biological probe which is adapted to interact with a different biological target sample, said plurality of sets of sites formed on said substrate; and
wherein said identifier identifies each of said plurality of biological probes.

4. The micro-array apparatus of claim 1 wherein said first set of sites are formed on said substrate by at least one of the following:
photolithography, ink jet printing, silk screening, liquid dispensing.

5. The micro-array apparatus of claim 1 wherein said set of sites are formed on said substrate by coating said substrate with a composition including a set of biological probe modified micro-spheres immobilized in a coating containing a gelling agent or a precursor to a gelling agent, said set of micro-spheres containing an optical bar code generated from at least one colorant associated with said biological probe, wherein said biological probe is adapted to interact fluorescently/chemiluminescently with fluorescently/chemiluminescently labeled biological target sample.

6. The apparatus of claim 1 wherein said identifier includes one or more of the following information: how the micro-array was manufactured, what specific materials it contains, how the micro-array can be read, application specific information, patient, technician or user information.

7. The apparatus of claim 1 wherein said at least first set of sites are formed on a region of said substrate and wherein said identifier is located on said substrate outside of said region.

8. The apparatus of claim 1 wherein said identifier is located on one of the front or back of said substrate.
